# EUROPEAN PATENT SPECIFICATION

(11) **EP 1 035 837 B1**
(45) Date of publication and mention of the grant of the patent: **09.04.2003**
(21) Application number: 98959011.2
(22) Date of filing: 08.12.1998
(51) Int. Cl.: A61K 9/127

(54) **LIPID COMPOSITIONS AND THEIR USE**
LIPIDZUSAMMENSETZUNGEN UND DEREN VERWENDUNG
COMPOSITIONS LIPIDIQUES ET LEUR UTILISATION

(30) Priority: 08.12.1997 GB 9725946
(43) Date of publication of application: 20.09.2000
(73) Proprietor: PHARES PHARMACEUTICAL RESEARCH N.V., Curacao Netherlands Antilles (AN)
(72) Inventor: LEIGH, Steven, Warlingham, Surrey CR6 HJ (GB)
(74) Representative: Schreiber, Wolfgang F.
(86) International application number: GB9803658
(87) International publication number: WO99029301

(56) References cited:
- EP-A- 0 648 494
- EP-A- 0 649 660
- WO-A-88/07871
- WO-A-98/58629
- DE-A- 19 520 659
- DE-C- 3 613 799

## Description

### INTRODUCTION

The present invention relates to methods for preparing lipid compositions, e.g. compositions that can be applied to the mucosa as soothing, protective or lubricating agents or for carriers of medicaments in molecular dispersion.

### PROBLEMS IN THE DELIVERY OF ANTI-FUNGAL AGENTS AND OTHER HYDROPHOBIC MEDICAMENTS

Yeasts, normally reside harmlessly on or in the human host, but on occasion, they can take advantage of the host's debilities and disorders to cause infections of a remarkably wide range of moist membraneous tissues. Such infections are usually described under the generic name candidiasis. Most common candidiasis infections are superficial lesions, especially infections of the mucous surfaces of the mouth or vagina. Oral and vaginal forms of candidiasis are commonly known as 'thrush'. Such ailments can normally be effectively treated with conventional topical antifungal preparations.

With the increasingly widespread use of antibiotics, immuno-suppressant and cytotoxic drugs, however, these normally relatively low-level infections can become much more serious. This is particularly the case for the increasing number of patients with diseases such as acquired immune deficiency syndrome, where the normal immunological protection of the patient is severely compromised. The low bio-availability of the active ingredients in conventional formulations means that these are generally employed sub-optimally and therefore there is an urgent need for effective delivery systems that are more patient compliant and convenient to use.

There is much current research into more efficient mucosal drug-delivery systems for treating fungal infections. The main problem lies firstly, in the nature of most anti-fungal agents and secondly, the area to be treated. Antifungal compounds are mostly lipophilic molecules with very low aqueous solubility which limits the release of drug to the surrounding milieu. This raises considerable problems concerning the choice of a suitable carrier in which the active ingredient can be formulated in a readily available molecular dispersion and thereafter retain a sufficient concentration locally in the presence of natural secretions.

A great deal of effort has been directed at tackling these two problems by the expedient of (a) employing solvents to solubilise the compound or (b) increasing muco-adhesion to prolong contact and release of the active ingredient to the mucosal surfaces. Improved muco-adhesion can be obtained by using water-soluble hydrocolloids (e.g. carboxymethylcellulose), polymers (e.g. carboxyvinyl co-polymers) and natural gums (e.g. sodium alginates) to form viscous gels which are applied to the mucosal surfaces. Alternatively, the hydrocolloids are simply dispersed in a non aqueous base. On exposure to water, these materials swell and confer some muco-adhesive properties to the base.

These approaches, whilst prolonging contact and increasing the potential for the transfer of active ingredient to the mucosal membranes still offer limited benefit because of the lipophilic and low aqueous solubility of most anti-fungal compounds. In reality, only a small amount of the drug is available in aqueous molecular solution for absorbtion, and the reservoir of undissolved drug is poorly mobilised. Ethanol and other organic solvents are commonly used in small amounts in the formulations to improve solubility. The efficacy of such preparations for topical use is however restricted by the tendency for the saliva and other body secretions to precipitate dissolved compounds. For adequate solubilisation, larger quantities of organic solvents and/or surfactants are therefore required. This in turn can create problems because the amount of active compound released from such formulations may not be sufficient due to unfavourably high partition coefficients. Furthermore, to be useful, the formulation should contain components that are non-toxic, non-sensitising and non-irritant, especially on inflamed and sensitive mucosal membranes. Preferably, it should have components that are naturally compatible with mucosal surfaces. In practice, most solvents and artificial surfactants that can effectively solubilise poorly soluble, lipophilic compounds fall short of these requirements. An ideal formulation would be a natural carrier that remains in the milieu around the infection, and delivers the drug in molecular solution.

Amphotericin is regarded to be one of the most potent antifungals. Few instances of resistance to amphotericin have been reported. However, its nephrotoxicity generally restricts its use in the topical treatment of mucosal fungal infections, where it is given as a gargle or a lozenge. It is administered intravenously only in serious systemic infections that do not respond to first line antifungals.

The use of liposomes to trap water insoluble/lipophilic molecules and thus allow higher, more effective doses to be given has been extensively explored. One reason for the reduction in toxicity of amphotericin in lipid carriers may be due to an association with the lipid, thereby reducing the amount of free drug which is more toxic. The translation of these benefits into practice to enable the wider and more effective use of amphotericin has, however, proved more difficult.

As detailed below, a number of approaches to liposome/lipid entrapment of amphotericin for the intravenous treatment of systemic fungal infections have been disclosed. These approaches, however, have proved too expensive for routine and widespread use because of their requirement for expensive lipids. In addition the manufacturing costs are high, requiring large volumes of solvents, evaporation, lyophilisation and specialised processing equipment. Therefore, there would be considerable benefits in reducing the complexity and costs in allowing amphotericin (and other lipophilic compounds) to be used more routinely in the treatment of diseases.

EP-A-0317120 claims a composition and method for solubilising a poorly soluble amphiphilic drug e.g. amphotericin, by forming a complex with a charged lipid, phosphatidyl glycerol (PG) in acidified organic solvent at a pH between 1 and 3. The complex can be incorporated into liposomes, which may be freeze-dried for long-term storage. The freeze dried composition is re-hydrated with aqueous buffer before administration. This method is claimed to give high lipid-amphotericin association, allowing higher doses of drug to be given intravenously.

US-A-5180713 also claims a freeze dried amphotericin composition prepared from lyophilising a liposome-amphotericin suspension having a predetermined size distribution between 0.2 to 0.5 micron in the presence of a stabilising agent eg a disaccharide, trehalose. The inclusion of trehalose is claimed to preserve the size of the liposomes on reconstitution with aqueous medium, thereby allowing long term storage of the freeze dried preparation.

EP-A-0282405 describes methods and compositions for non-liposomal lipid complexes formed from associations between toxic hydrophobic drugs such as amphotericin B and combinations of dimyristoylphosphatidylcholine (DMPC) and dimyristoylphosphatidyl glycerol (DMPG) in about 7:3 mole ratio.

EP-A-0 648 494 is concerned with a rapamycin formulation for oral administration. A typical method for the production of the formulation consists of the following method steps:
- rapamycin is first dissolved in a non-aqueous solvent (N,N-dimethylacetamide);
- in a second step polysorbate 80 is added;
- finally the composition is adjusted to the final volume with Phosal 50 PG® lecithin and propylene glycol.

While the method of EP-A-0 648 494 may produce a solution or emulsion of rapamycin with an amphiphilic membrane forming lipid, the drug is not in molecular dispersion in bilayers of membrane lipids. The method as disclosed in EP-A-0 648 494 requires an additional surfactant (polysorbate) to carry the biologically active component It is to be noted that polysorbates are not bilayer forming membrane lipids. In fact it is known that polysorbates often even destroy bilayers.

The prior art contains a large number of reports on the clinical benefits of lipid-amphotericin associations. The smaller number of disclosures concerning compositions and methods of preparation all require essentially charged lipids with high phase transition temperatures e.g. di-palmitoyl phosphatidylglycerol (DPPG), combined with neutral lipids e.g. di-myristoyl phosphatidylcholine (DMPC) and di-palmitoyl phosphatidylcholine (DPPC). The negatively charged lipid forms a complex with ionised amphotericin at low pH.

### USE OF THE PRESENT LIPID COMPOSITIONS TO SOLUBILISE WATER-INSOLUBLE AND LIPOPHILIC COMPOUNDS

The present invention describes a novel approach to the problems of using membrane lipids for the solubilisation and preparation of lipid based compositions containing water insoluble and lipophilic compounds generally, and anti-fungal agents in particular. The method disclosed is simple, effective and inexpensive to put into practice. Furthermore it has the advantage of employing membrane lipids or mono-acyl derivatives thereof that are compatible with natural membrane surfaces, and are not harmful. Advantageously, the membrane lipids used in the invention confer improved chemical and physical stability to the active compound and allow extended storage under appropriate conditions. The resultant compositions surprisingly show enhanced biocidal activity when tested against conventional formulations of the same compound at similar concentrations.

The invention provides a method for preparing a composition as defined in claim 1, said method including the steps set out in that claim.

The present method enables a lipophilic compound e.g. a fungicide to be incorporated in solution or dispersion in a lipid, said lipid being in the form of solvated bilayers in a generally anhydrous organic medium. It should be understood that the term "anhydrous" as used herein, does not exclude the presence of small amounts of water and those quantities of water which are carried in ingredients such as ethanol and glycerol and which are difficult or expensive to remove completely.

The present method enables lipophilic biologically active materials to be incorporated into membrane lipid in a form which converts into liposomes, micelles or related structures with high entrapment on addition or exposure to aqueous medium. It should be emphasised-that the formation of liposomes is not an essential attribute of the composition formed. In some cases, depending on the nature of the active compound and/or the choice of lipid used, the compositions may result in formation of one or more lipid structures such as micelles or mixed micelles. The type of lipid structures formed could also depend on the extent of dilution, diluting medium and presence of other components encountered in different applications. The important feature is for the lipophilic compound to remain substantially solubilised by the lipid when the compositions are diluted with aqueous fluids.

One principal concern of the invention is to provide compositions that can be applied directly to mucosal membrane surfaces. Conversion from bulk bilayered structure to ultimately, discrete lipid structures takes place in-situ, utilising endogenous body fluids or secretions. Alternatively, the conversion may take place on simple dilution with external aqueous medium, prior to use. In both cases, high concentrations of the bioactive compound will be solubilised in the resultant lipid structures. Therefore, delivery of the active compound to the mucosal infection may be greatly enhanced.

The compositions, may be applied to the places indicated below for the purposes indicated below.
(a) oral, buccal mucosa for soothing mouth ulcers;
(b) vaginal mucosa for soothing, protection or lubrication;
(c) rectal mucosa for the relief of haemorrhoids;
(d) a stoma for a protection and/or lubrication;
(e) a surgical device or glove as a lubricant;
(f) gastric mucosa.

They are resistant to microbial growth and may be used without added preservative. They preferably have a pH of 5 - 7.5. The compositions may be used as such, or they may be mixed with water or aqueous liquid prior to use e.g. to produce a rinse or gargle.

The compositions made according to the present method comprise at least one membrane lipid and/or micelle-forming lipid dispersed in an anhydrous hydrophilic medium consisting of a mixture of a first liquid (e.g. ethanol) for forming a molecular dispersion of the active ingredient within the lipid component and a second liquid (e.g. glycerol) miscible with the first organic liquid for precipitating the membrane lipid as bilayers. At least one of the first and second aqueous liquids may be water-miscible and preferably they are both water-miscible. Active ingredients (for example hydrophobic biocidal agents, steroids, cytotoxic compounds, immunomodulators or immunosuppressants) are partitioned between these lipid bilayers and the hydrophilic liquid phase. The compositions will normally be in the form of a gel, the viscosity of which is dependent on the relative proportions of constituents.

Compositions made by the present method may comprise four main components:
(a) at least one membrane lipid or mixture of a membrane lipid and a micelle-forming lipid;
(b) one or more active ingredients;
(c) at least one pharmaceutically acceptable solvent for the lipid; and
(d) at least one hydrophilic organic liquid that can cause precipitation of lipid bilayers.

Any membrane lipid, or combination of membrane lipids capable of forming lipid bilayers may be used as component (a). Preferred lipids for use in the present invention include egg yolk lecithin, soybean lecithin, phospholipids (e.g. phosphatidylcholine (PC), phosphatidylethanolamine (PE), phosphatidylglycerol (PG), phosphatidic acid (PA) and sphingomyelin), glycolipids, cerebrosides, gangliosides and their lyso-derivatives e.g. enzyme-modified monoacyl derivatives. Where the use dictates, mixtures of modified lipids such as hydroxylated and acetylated phospholipid may also be used. For specialised use, such as in drug-targeting applications, the lipids may also be in the form of derivatives (e.g. with polyoxyethylene chains) to make the vesicles more robust. Monoacyl lipids which may be derived from glycolipids, sphingolipids or other micelle forming lipids and may be derived from natural plant, animal or microbacteriological sources, or they may be synthesized or partly synthesized e.g. polyethylene glycol (PEG) derived monoacyl phospholipids e.g. pegalated monoacyl phosphatidyl ethanolamine. Preferably, the lipid comprises two or more membrane lipids. Mixtures of lipid commonly known as lecithins are preferred. They may be natural or synthetic, hydrogenated, partially hydrogenated or unsaturated. They may be of animal, enzyme modified or plant origin. In practice, commercial mixtures of lecithins and enzyme modified lecithins which contain at least 95% of total lipids, of which the major component is at least 40% phosphatidylcholine, are preferred. The amount of lipid used depends on the active ingredients and should be sufficient to ensure that it is in molecular dispersion. In general about 50% is the highest amount that should be required, although larger amounts can be used where necessary. The usual range is between 10% to 30% of phospholipid.

The invention enables improvements to be brought about in the bio-availability and efficacy of a large number of biologically active materials including medicaments, hormones, vaccines, peptides, vitamins and related materials of medical and biological interest such as antioxidants, marker compounds and flavouring materials. The invention is particularly useful for solubilising water insoluble and lipophilic bioactive compounds for the topical treatment of the skin and of superficial infections of the moist cutaneous areas of the body involving the oral mucous membranes, upper respiratory tract, and vagina. The compositions are especially suitable for local application to mucosal membranes to treat conditions which respond to topical medication with anti-inflammatory, biocidal and cytotoxic agents or immunomodulating agents (vaccines). In a specific embodiment, the invention is a carrier for anti-fungal compounds.

The active ingredient (b) can be any of a wide-range of biologically active lipophilic materials including medicaments, hormones, vaccines, peptides, vitamins immunosuppressants (e.g. cyclosporin or other cyclic peptides) or immunomodulators (vaccines). Alternatively, it could be any other lipophilic material of medical and biological interest such as antioxidants, marker compounds and flavouring materials. The amount of lipophilic compound carried in the composition can be up to 10% , but typically it is between 0.1% to 5% and preferably 0.1-2.5% w/w. For very potent compounds, amounts less than 0.1% are often used.

Examples of the component (b) include:
(i) an anti-fungal agents for *Candidiasis* for application to the oral mucosa or vagina;
(ii) an anti-viral agents for application to the oral mucosa or vagina;
(iii)an anti-inflammatory agents for application to the oral, rectal or vaginal mucosa.

The biologically active compound may, for example be a polyene macrolide. (e.g. nystatin or amphotericin B), or an imidazole derivative (e.g. clotrimazole or miconazole), or it may be 5-fluorocytosine, acyclovir, triamcinalone or another steroidal anti-inflammatory agent).

The biologically active compound (b) may be added to the solution or dispersion of the lipid (a) as a micrconised powder e.g. a powder produced by milling the biologically active compound in a colloid mill,
Component (c) is an anhydrous preferably water-miscible organic liquid which is a solvent for the lipid and in most cases for the lipophilic active compound. In general, the amount of compound (c) should not exceed that required to dissolve the compound and lipophilic compound and allow the formation of a molecular dispersion of the active ingredient. Ethanol is the preferred component but ethanol-free formulations may be required for some purposes and other aliphatic alcohols, e.g. methanol, propanol, and isopropyl alcohol, or other suitable solvents (e.g. propylene glycol, diethylene glycol monoethyl ether (Transcutol) and propylene glycol carbonate), or mixtures of such solvents, may be used in those applications where their use is permitted. The broad range of component (c) that can be used is between 2.5 to 25 wt%. The preferred range is 2.5 to 10 wt%. The most preferred range is 5 to 7.5 wt%.

Component (d) is a second anhydrous organic liquid, preferably miscible with water and component (b), which is of a nature, and is present in an amount, to cause the membrane lipid (or lipids) in the preparation to form bilayers. Suitable liquids are glycerol, which is preferred on account of its absence of toxicity, or propylene glycol, but again other hydrophilic liquids for example the polyethylene glycols, glycofurols and propylene glycol derivatives may be used in those applications where their use is permitted. It will be noted that, depending on the other materials used, propylene glycol can be component (c) or component (d). The broad range of component (d) is between 30 to 90 wt%, the preferred range 40 to 90 wt% and the most preferred range 50 to 80 wt%.

To avoid unacceptably high concentrations in the final composition, and the possible requirement of complicated solvent removal steps, component (c) should be used in relatively low concentration. The amount of ethanol required to solubilise the lipid is typically about 25% w/w based on the weight of the lipid. Preferably the amount by weight of ethanol (or any alternative solvent used as component (c)) should not exceed the amount by weight of lipid. Complete dissolution of the lipid component in this first stage of preparation although desirable, is not always possible or indeed necessary, in order to achieve the formation of a molecular dispersion of the active ingredient in the final composition.

The second stage of the preparation consists of the addition of component (d) in order to precipitate the lipid as bilayers. The concentration of component (d) is, in most cases, not critical and is dictated mainly by the desired rheological properties of the preparation. It should, however, be present in sufficiently high concentration to ensure that all, or by far the greater part of the lipid, is in a bilayer configuration.

In some cases, where the active ingredient has significant solubility in mixtures of components (c) and (d), and exposure of the lipid and/or the active ingredient to high temperatures for prolonged periods of time must be avoided, complete dissolution of the active ingredient is unnecessary when the composition is initially formed and the preparations will clear on standing. The efficiency of incorporation of the active ingredient under these conditions, however, is strongly dependent on the degree of subdivision of the active material. The active ingredient may be of particle size 1 to 10 µm at the time of incorporation, or it may be incorporated at larger particle size and the composition may then be treated in a colloid mill to bring about molecular dispersion.

Similarly, in compositions containing high concentrations of active ingredient, precipitation of some of the active ingredient in the form of small particles may occur on the addition of component (d). This, however, is often reversible on standing.

In presentations such as antifungal gels, it is beneficial to prolong contact between the composition and the underlying lesion. In such cases, small amount of a hydrophilic colloid such as a hydrocolloid, polymer or a natural gum can be blended into the preparation. Suitable materials are water-soluble but insoluble in the non-aqueous composition. Alginates e.g. sodium alginate, carrageenan, cellulose derivatives, e.g. hydroxypropyl cellulose and a copolymer of N-vinyl-2-pyrrolidone and vinyl acetate (Plasdone) have been successfully employed. On application, the gums hydrate in aqueous fluid and confine the composition to the site of application, thereby allowing extended conversion into liposomes or other lipidic particle structures. Alternatively to provide compositions that have protective properties, larger amounts of the hydrocolloid may be required and the gel base may be used with or without a medicament. In general we have found that amounts of polymer, hydrocolloid or natural gum incorporated should be 0.5 to 10 wt.% based on the total weight of the composition, preferably 1-10 wt% and more preferably 2 to 5 wt.%. Below 0.5 wt.% there is only slight improvement in adhesion, whereas above 10 wt.% the material becomes glutinous and is difficult to handle.

The substantially non-aqueous compositions advantageously have a pH of 5-7.5.

Formulations of this invention can be reliably and economically produced on a large scale and can be stable for extended periods. They can be produced under aseptic conditions or, on warming to a temperature of about 45?C, or may be sterilised by aseptic filtration. They may be packed in tubes or in pump-type dispensers.

The invention, and in particular, its application to the formulation of anti-fungal compositions, is illustrated in the accompanying examples.

### EXAMPLE 1

### Miconazole and Clotrimazole Formulations

Anti-fungal gels were made from the following formulations:

| | |
|---|---|
| *Phospholipids ( 45% phosphatidylcholine-PC) | 20% |
| Absolute ethanol | as indicated |
| Antifungal drug | as indicated |
| Glycerol B.P. | as indicated |

| | |
|---|---|
| * Epikuron (Lucas Meyer) | |

Approximately 10% of the total lipid and all the ethanol were weighed into an amber glass jar and mixed until all the lipid had dispersed. The antifungal drug was added to the lipid/ethanol mixture and further mixing was carried out to break up the drug particles. The remainder of the lipid was added and mixing was continued until all the lipid had been dispersed. The glycerol was then added and stirring was continued until a homogenous gel was obtained. It was clear and translucent with a sweet pleasant taste and could be applied directly to the mucosa. From about 1% to 10% or more, but preferably about 2% to 5% of a hydrophilic polymer or natural gum can be dispersed in powder form to render the preparation muco-adhesive. Compositions utilising lipid blends or lecithins which contain 45% PC, form liposomes on conversion with water. The drug is associated with the liposomes at very high levels, ie greater than 90%.

Miconazole (1-[2-dichloro-β-[2,4-dichlorobenzyl) oxyphenethyl]imidazole mononitrate is a white crystalline powder that is very slightly soluble in water and very soluble in ethanol. It has a wide antifungal spectrum and possesses some antibacterial activity. It is available from R W Unwin & Co. Ltd. Clotrimazole[1-(o-chloro-a,a-diphenylbenzyl)imidazole] is a white crystalline powder that is insoluble in water and soluble in ethanol. It has a wide antifungal spectrum and possesses some antibacterial activity. It is available from Fabbrica Italiana Sintetici.

The preparation of the pro-liposome miconazole and clotrimazole gel was as described above. Early formulation work was carried out to find:
i) A gel formulation with enough ethanol to dissolve the miconazole and clotrimazole giving the gel a translucent appearance, but with sufficient ethanol as to obtain an optimum viscosity profile (Table 1).
ii) A gel formulation with not too much miconazole or clotrimazole to induce crystallisation of the drug in the gel (Table 2).

5% ethanol in the gel was found to be most suitable for both gel viscosity and drug crystallisation. At this level of ethanol, miconazole drug crystallisation was not seen in 1% and 2.5% miconazole gals. Drug crystallisation was seen in the 1% and 2.5% clotrimazole gels, but as the 1% clotrimazole showed much less drug crystallisation, it is expected that gels with just less than 1% clotrimazole would show no drug crystallisation at all.

The % encapsulation of the antifungal drugs miconazole and clotrimazole was determined by filtration of the liposome dispersion and by HPLC analysis of the unfiltered and filtered solutions for drug content. The principle behind the filtration technique is that the liposomes (with encapsulated antifungal). can be passed through 200 nm filters, while unencapsulated drug particles remain too large to pass through the filters. Pro-liposome gels were prepared, containing 2.5% and 1% miconazole and 1% and 0.5% clotrimazole. The % drug encapsulation into liposomes are shown in Table 3. It was found that both the miconazole and clotrimazole gels showed high levels of liposomal encapsulation. The % encapsulation of drug would be expected to decrease if crystallisation occurred in the gels with time.

Miconazole and clotrimazole gels were prepared for long term stability studies with drug concentrations equal to, or less than corresponding proprietary preparations. Samples were stored in sealed glass containers, protected from light, at 4oC, 25°C, and 40°C. The content of drug in the gels was assayed by HPLC analysis to evaluate the extent of drug degradation. The stability of both miconazole and clotrimazole, in the pro-liposome formulations was dependent on the drug concentration. The higher the concentration, the lower the rate and extent of degradation. Drug solubility in the base formulation is known to limit the optimal extent of drug concentration, thus the formulation remains a balance between stability and concentration. The crystallisation of drug in the gel was measured by measuring liposomal encapsulation over time.

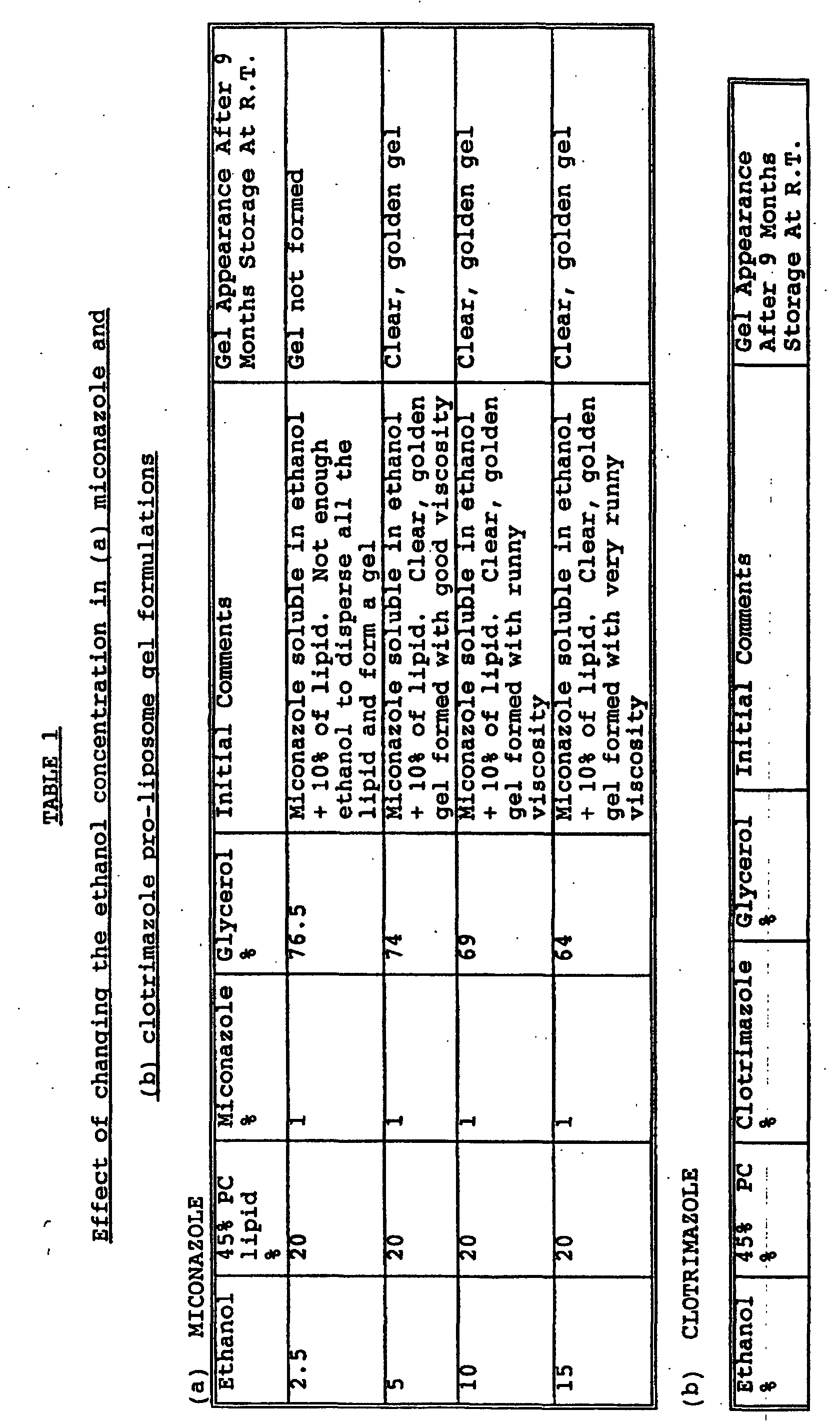

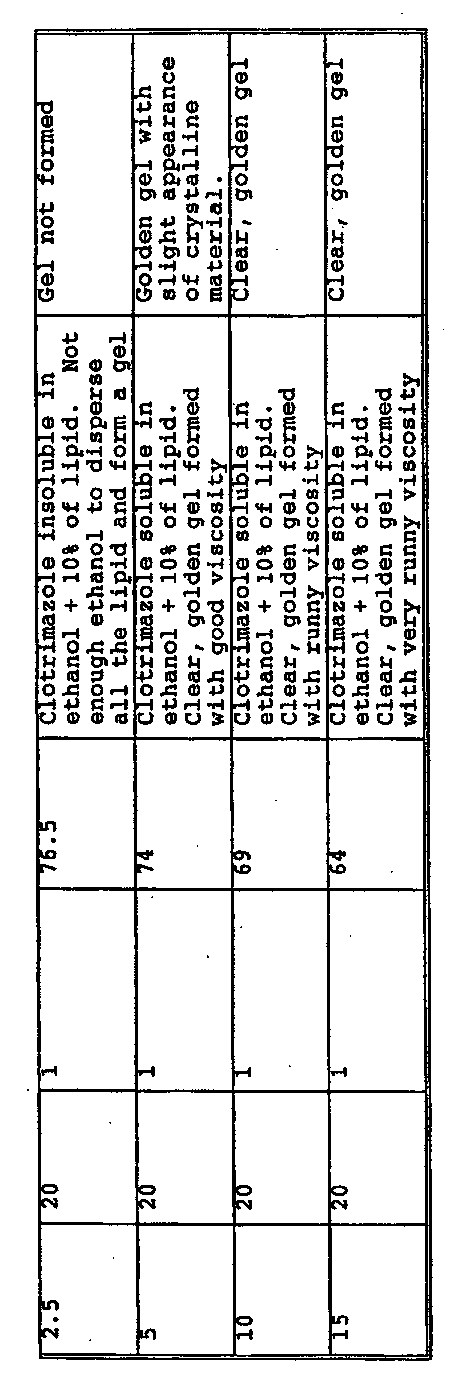

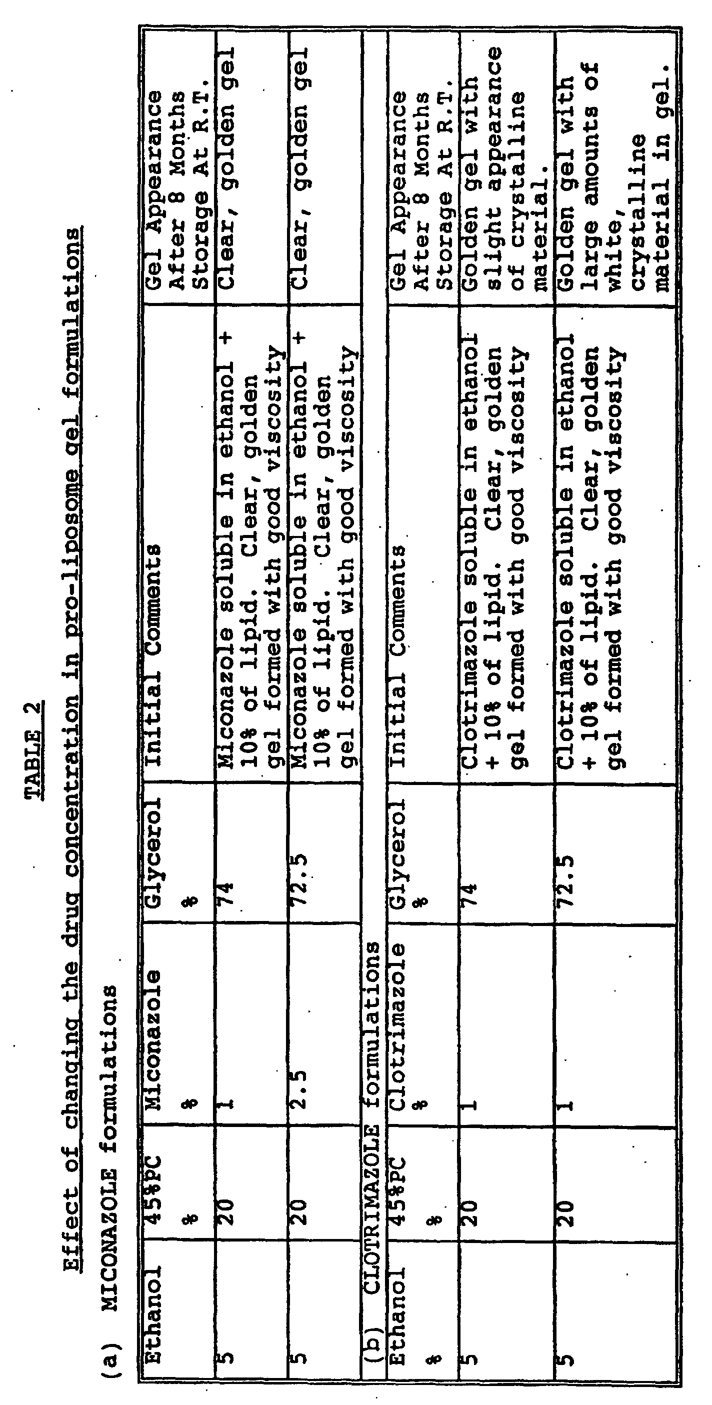

**TABLE 3**

| The % encapsulation of antifungal drugs into liposomes from pro-liposome gels | | |
|---|---|---|
| | % Encapsulation of Antifungal | |
| Sample | T = 5 months | |
| 1 % Miconazole Gel | 4°C | 97.4 % |
| | 25°C | 102.2 % |
| | 40°C | 99.4 % |
| 2.5 % Miconazole Gel | 4°C | 100.4 % |
| | 25°C | 97.8 % |
| | 40°C | 97.5 % |
| 0.5 % Miconazole Gel | 4°C | 98.3 % |
| | 25°C | 96.8 % |
| | 40°C | 98.5 % |
| 1 % Clotrimazole Gel | 4°C | 94.4 % |
| | 25°C | 96.3 % |
| | 40°C | 97.2 % |

### EXAMPLE 2

### Gels containing Amphotericin B

Using the method of Example 1, pro-liposome gels were made with various concentrations of amphotericin B. The % encapsulation of the amphotericin B into liposomes (in 0.1% suspensions of the gel formulations) was measured at 3 days, 1 month, 3 months and 6 months after pro-liposome preparation. The gels were stored at 4°C ;in sealed containers, protected from light (Table 4). It was found that amphotericin B showed high levels of liposomal encapsulation with lipid: drug ratios in the range 20:1 - 200:1, and the level of encapsulation level changed relatively little on long term storage.

**TABLE 4**

| The % encapsulation of amphotericin B into liposomes from a pro-liposome on long term storage at 4°C. | | | | |
|---|---|---|---|---|
| Sample | % Encapsulation Of Amphotericin B | | | |
| | T = 3 days % | T = 1 month % | T = 3 months % | T = 6 months % |
| 1 % Amphotericin B Gel (20:1, lipid:drug) | 103.2 | 92.2 | 95.4 | 89.0 |
| 0.5 % Amphotericin B Gel (40:1, lipid:drug) | 97.0 | 102.9 | 93.4 | 94.6 |
| 0.25 % Amphotericin B Gel (80:1, lipid:drug) | 97.0 | 96.4 | 95.8 | 94.6 |
| 0.1 % Amphotericin B Gel (200:1, lipid:drug) | 88.5 | 95.5 | 94.8 | 90.5 |

### EXAMPLE 3

### Gel containing amphotericin B and buffer

A pro-liposome gel containing amphotericin B was formulated as follows:

| | |
|---|---|
| *Phospholipids (90% PC) | 20% |
| Absolute ethanol | 5 % |
| Amphotericin B | 1 % |
| Glycerol B.P. | 73.9 % |
| Tris base (Sigma) | 0.1 % |

| | |
|---|---|
| *Epikuron (Lucas Meyer) | |

The gel was made by dispersing the lipid, 90% of which is phosphatidylcholine, and ethanol into a vessel which was sealed and left for 24 hours at 25°C until the lipid had dispersed. The amphotericin B was then added to the lipid/ethanol mixture and homogenised. Tris base for buffering the gel to approximately pH 6.5 was dissolved in the glycerol by heating them together at 60°C for about 30 minutes, after which the solution was cooled to room temperature. The glycerol solution of Tris base was stirred into the lipid mixture until a homogeneous bilayered gel formed. A similar preparation can be made using a lipid mixture containing 45% PC.

Amphotericin B with an average particle size of 1-5µ was found to be well incorporated into the gel. It showed near 100% encapsulation into liposomes formed from the pro-liposome by addition of water, whereas incorporation of coarser particles was found to be much less efficient. The selected content of Tris base provides good stability for the lipid whilst increasing the stability of the amphotericin B compared to a gel containing no buffer.

### EXAMPLE 4

| | |
|---|---|
| *Phospholipids (70% PC) | 20% |
| Ethanol | 5 % |
| Triamcinalone | 0.1 % |
| Glycerol | 72.9% |
| Sodium alginate | 2% |

The method of Example 3 was followed to prepare the bilayered gel. 2% sodium alginate powder was uniformly dispersed in the composition, giving it improved mucoadhesive properties and a slightly more opaque appearance.

### EXAMPLE 5

### Microbiology

Microbiological work was carried out to elucidate two fundamentally different properties of the pro-liposome gel formulation;
- To assess the intrinsic antimicrobial activity of the gel base (without antifungal drug and without dilution to a liposomal dispersion) as a measure of whether the gel base alone would inhibit or support microbial growth on long-term storage, or whether it would need to contain additional antimicrobial preservatives.
- To assess the antifungal activity of the pro-liposome gel formulation (with antifungal drug, diluted to an aqueous liposomal dispersion) compared to an equivalent aqueous suspension of the antifungal drug.

### 1. Gel Base Microbial Challenge

The antimicrobial efficiency of a pharmaceutical preparation is assessed by a simple challenge test (Based on Appendix XVI C BP 1988 P. A200 - P. A202). The following organisms were used in the test: *Aspergillus* niger NCPF 2275, *Escherichia coli* NCTC 1487, *Candida albicans* NCPF 3179, *Pseudomonas aeruginosa* B15 and *Staphylococcus aureus* NCTC 4135.

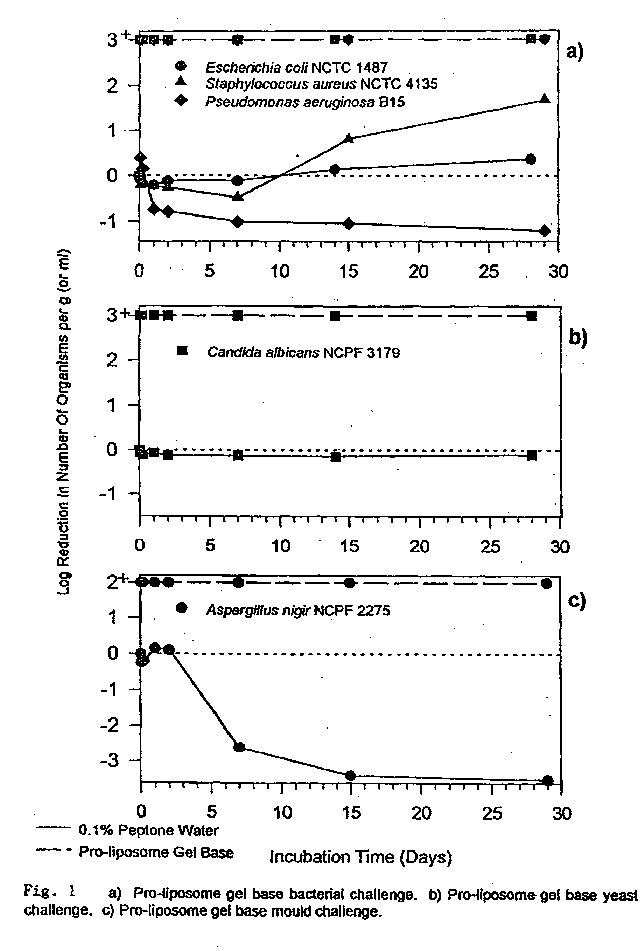

For the pro-liposome gel base, 10g placebo samples prepared in accordance with Example 1, without the inclusion of the antifungals, were inoculated with micro-organisms to give an inoculum of 1 x 10⁶ bacteria or yeast per g of sample or 1.4 x 10⁴ mould per g of sample (inoculum volume 1 % of sample). Control samples were prepared in the same way using 0.1 % peptone water (for bacteria and yeasts) or 0.1 % peptone water + 0.5 % Tween 80 (for moulds). The inoculated samples were stored at 20°C for 1 month. At regular intervals, 1 g sub-samples were removed, and diluted appropriately to determine the number of remaining microorganisms in the sample. The results are shown in Figure 1 which shows the result of challenging the gel base with (a) bacteria, (b) yeast and (c) mould.

For bacteria and yeasts, the number of organisms recoverable from the gel is instantly reduced by a factor of at least 10³. For the mould, the number of organisms recoverable from the gel is instantly reduced by a factor of at least 10². It is thought that this surprisingly effective antimicrobial activity is the consequence of both the microcidal effect of the ethanol and the osmotic potential of the glycerol. It was an unexpected bonus and as a result, the pro-liposome gel base alone is expected to have acceptable preservative properties for many uses as a topical or oral pharmaceutical preparation, without the need for additional preservatives in the gel base (i.e. the pro-liposome gel base formulation may pass the BP microbial challenge test). Most preservatives used to prevent microbial spoilage are sensitisers. Therefore preparations that have natural preservation properties without the need for added antimicrobials are preferred.

### 2. In Vitro Antifungal Activity

The antifungal properties of the pro-liposome gel (with antifungal) was assessed using two methods. The first, the cup-plate diffusion assay, examines antifungal activity by diffusion through a solid medium. The second, evaluates antifungal activity in a liquid medium. The tests were both carried out to compare the efficacy of the liposomal antifungal preparations according to the invention, to that of equivalent aqueous suspensions of the antifungal.

### a) Cup-Plate Diffusion Assay

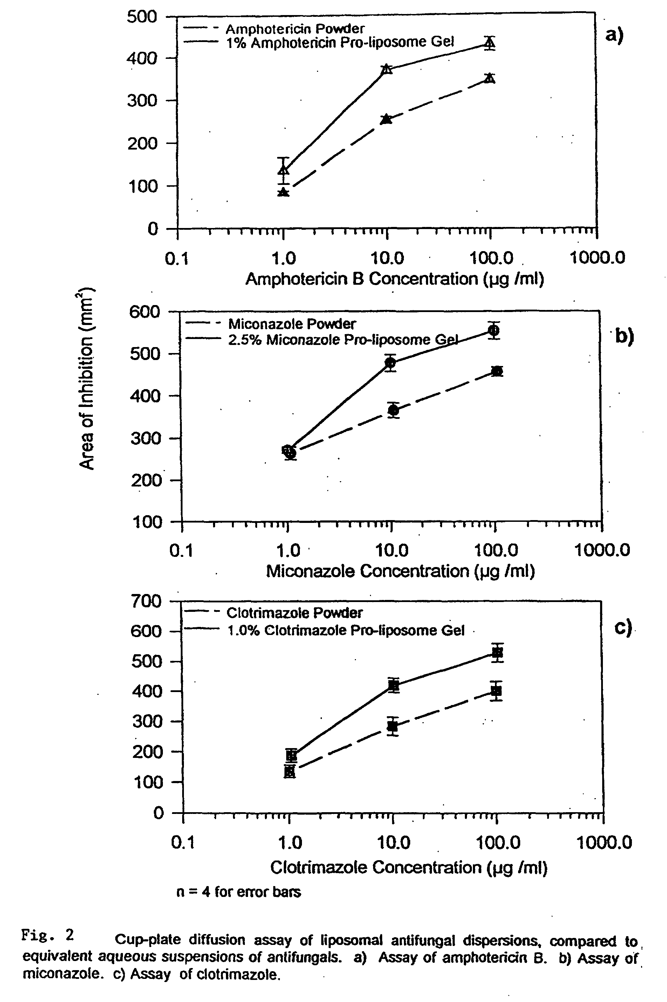

The pro-liposome gels containing 1% miconazole, 2.5% clotrimazole and 1.0% amphotericin, prepared from Examples 1, 2 and 3 respectively, were mixed and diluted appropriately, in aqueous phosphate buffer to form liposomal dispersions containing the same concentration of drug as the aqueous suspensions (1, 10 and 100 µg/ml). A cup-plate diffusion assay was carried out using *Candida albicans*. Tryptone-soya agar plates were used that had been inoculated with *Candida albicans* NCPF 3179 to a final concentration 10⁶ viable cells per ml. Solutions were incubated in 5 mm wells for 2 hours at room temperature, followed by 18 hours at 37°C. The zones of growth inhibition of the *Candida albicans* were then measured (Figure 2) in comparison with equivalent aqueous suspensions of antifungals for (a) amphotericin B, (b) miconazole and (c) clotrimazole.

The liposomal formulations of the antifungal drugs, showed greater zones of inhibition than the aqueous antifungal suspensions. By comparison, the control gel base (without antifungal) showed no zone of inhibition. The larger zones of inhibition with the liposome preparations reflects the higher bio-availability of the antifungal drugs from this formulation.

### b) Growth Inhibition In A Liquid Medium

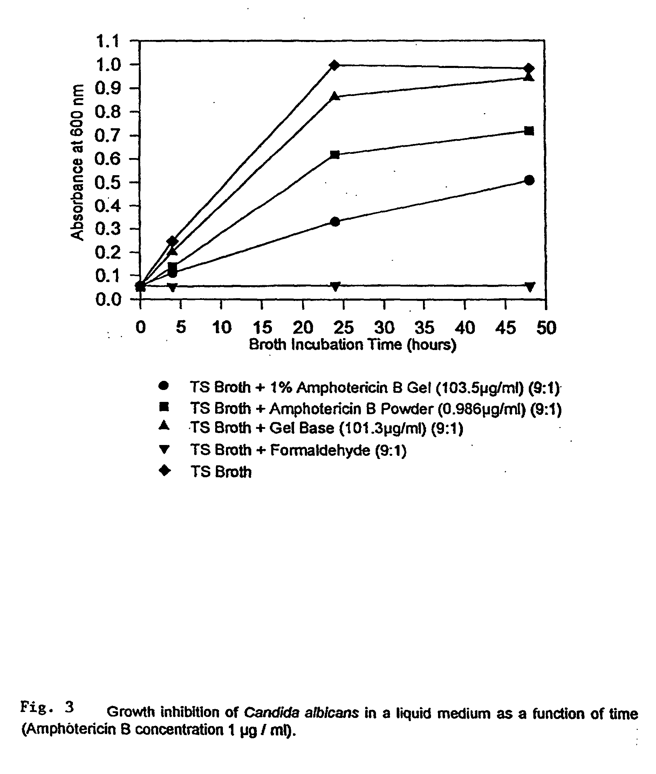

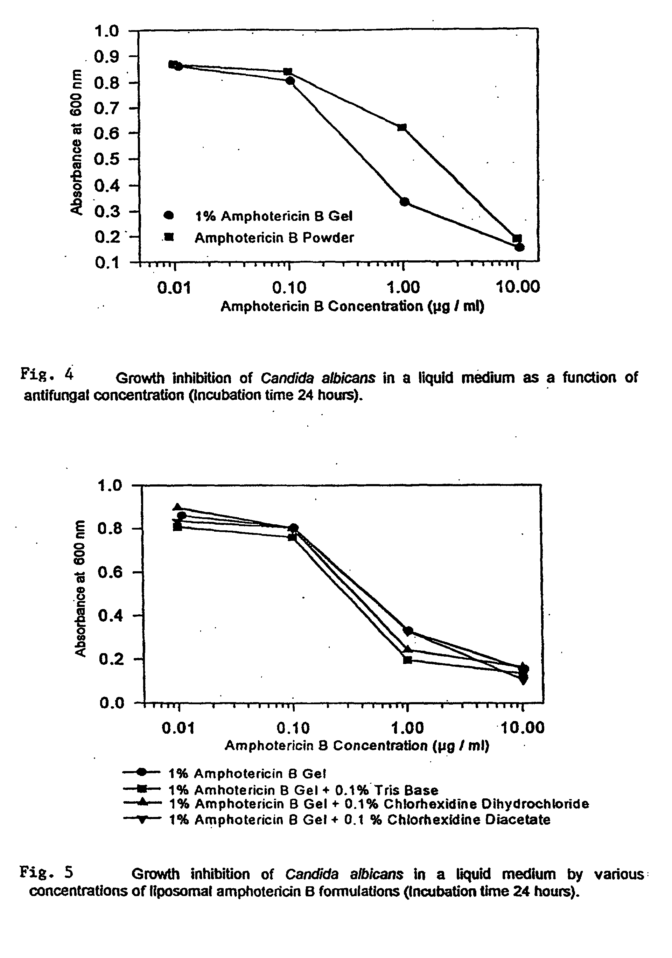

The pro-liposome gels were mixed, and diluted appropriately, in aqueous medium to form liposomal dispersions containing the same concentration of drug as aqueous suspensions. 1 ml samples were then mixed with 9 mls of tryptone-soya broth, innoculated with *Candida albicans* NCPF 3179 to a final concentration 10⁶ viable cells per ml. The optical density (at 600 nm) was then read at intervals as a measure of the growth (and growth inhibition) of the *Candida albicans.* Figure 3 shows the growth inhibition of *Candida albicans* in a liquid medium as a function of time (Amphotericin B concentration 1 µg/ml). Figure 4 shows the inhibition of *Candida albicans* in a liquid medium as a function of antifungal concentration (Incubation time 24 hours). Figure 5 shows the growth inhibition of *Candida albicans* in a liquid medium by various concentrations of liposomal amphotericin B formulations (Incubation time 24 hours).

As in the cup-plate diffusion assay experiment, the liposomal preparation was seen to be much more efficient at inhibiting the growth *Candida* compared to the aqueous dispersion. This emphasises the high bio-availability of liposomal amphotericin B and the ability of the liposomal preparation to act as a reservoir for the antimicrobial.

The *in vitro* tests indicate that the pro-liposome antifungal gels exhibit superior antifungal activity compared to the free antifungal drugs alone. If this property is extrapolated to an *in vivo* situation, then it is likely that the natural affinity of phospholipid for mucosa surfaces would further improve the retention and availability of the antifungal drug.

The pro-liposome formulation is ideal as a carrier for topical administration of antifungal drugs to mucosal surfaces. All the formulation components are pharmaceutically acceptable. The gel has a pleasant, sweet taste and is sufficiently viscous to allow it to be applied to the gums or mucosa if desired. Furthermore, on conversion to liposomes or other lipid structures (depending on the type of lipid used), the active compound remains in molecular solution, with a very high degree of entrapment, prolonging release of drug at the site of infection. The gel base alone does not support microbial growth and, using careful production conditions, the gel should have a very low initial microbiological burden.

The formulations containing amphotericin B disclosed in this invention, are likely to be stable at 25°C for several months and at 4°C for at least a year, enabling development of an improved, commercially viable pharmaceutical product with a good shelf life. A stable pro-liposome formulation which delivers relatively high doses to the intended treatment site should benefit to patients with Candida infections. The pro-liposome gel is easy and economical to produce, and shows much potential for improving the bio-availability of several different antifungal drugs. Furthermore, the presentation of a convenient and palatable gel should promote patient compliance.

### EXAMPLE 6

### Additional polymer and hydrocolloid materials

The method of Example 4 was repeated with 1 wt.% and 5 wt.% sodium alginate (Keltone HVCR) and with 1 wt.%, 2 wt.% and 5 wt.% each of carrageenan (Gelcarin GP37ANF), hydroxypropyl cellulose (Kucel HF) and a copolymer of N-vinyl-2-pyrrolidone and vinyl acetate (Copolyvidone; Plasdone S630). There was improved adhesion in each case compared to a corresponding formulation with no added polymer, and the improvement in adhesiveness was in the order sodium alginate > carrageenan >> Copolyvidone >> hydroxypropl cellulose. The gel containing 5 wt.% sodium alginate tested by adhesion to dialysis tubing [RS Manly (Ed), Adhesion in Biological Systems, Academic Press, N.Y. 1970, Chapter 10, J L Chen and G N Cyr, 'Compositions Producing Adhesion through Hydration'] produced a mean adhesion time of about 230 minutes, and 5 wt.% carrageenan produced a mean adhesion time of about 190 minutes. Microbiological studies showed that none of the polymers, at the levels used, materially reduced the antifungal activity of the amphotericin-containing pro-liposome gel which remained greatly superior to that of a commercially available amphotericin suspension used as a control.

### EXAMPLE 7

### Use of Alternative Solvents

The lipid of Example 1 was found to disperse in Transcutol (Gattefosse), lauryl lactate and isopropyl myristate at a solvent: lipid ratio of at least 1:1. The above solvents can mix into ethanol at certain concentrations, replace the ethanol used in other examples, but are not water miscible. Samples were then prepared in which amphotericin and lipid were dispersed in these solvents and in each case bright yellow opaque suspensions of the drug were obtained. Glycerol was added to portions of the well-dispersed samples to form gels whose viscosity may be controlled by the amount of glycerol added. Suitable formulations are as follows, the proportions being of lipid:solvent:amphotericin:glycerol.

| | |
|---|---|
| Transcutol | 20:20:1:59 |
| Lauryl Lactate | 20:30:1:49 |
| Isopropyl Myristate | 20:30:1:49 |

### EXAMPLE 8

### Ethanol-free amphotericin pro-liposome gel

A lipid (2g) which contains about 45% phospholipid of which 33% is phosphatidyl choline and about 10.5% is mono-acyl phosphatidyl choline and ethanol (0.5g) were placed in a glass tube and left at 40°C until the lipid had become dispersed in the ethanol. The resulting solution was cooled to room temperature and amphotericin (0.1g) was added. The resulting lipid mixture was subjected to ultrasound for 30 minutes. Separately TRIZMA base (TRIS, 0.02g) which is a buffer and glycerol (14.78g) were heated at about 200°C for about 30 minutes after which the base had completely dissolved. The glycerol solution was cooled to room temperature and a portion (7.4g) was mixed with the lipid mixture to produce an opaque mustard-coloured gel. The above formulation is ethonal free, as Example 7, and the presence of the mono-acyl phosphatlidyl choline promotes uptake of the additive.

## Claims

1. A method for preparing a composition comprising at least one lipophilic biologically active compound in molecular dispersion in bilayers of a substantially non-aqueous composition comprising at least one membrane lipid suspended in a hydrophilic medium, said method including the steps of:
dissolving or dispersing at least a portion of the membrane lipid in a first organic liquid which can dissolve of disperse the lipid;
adding the lipophilic biologically active compound to the solution or dispersion; and
adding a second organic liquid which is miscible with the first organic liquid and is of a nature and in an amount such that the membrane lipid or lipids is or are at least partly in the form of bilayers, at least one of the first and second liquids being water -miscible.

2. The method of claim 1, wherein the biologically active compound is added to the solution or dispersion as a micronised powder.

3. The method of claim 1 or 2, wherein the added biologically active compound is a polyene macrolide.

4. The method of claim 1 or 2, wherein the added biologically active compound is nystatin or amphotericin B.

5. The method of claim 1 or 2, wherein the added biologically active compount is an imidazole derivative.

6. The method of claim 5, wherein the added biologically active compount is a clotrimazole or miconazole.

7. The method of claim 6, wherein the added biologically active compound is 5-flourocystosine.

8. The method of any preceding claim, wherein there is added 0.1-2.5% w/w of the biologically active compound.

9. The method of claims 1 to 8, wherein there is added about 1% w/w of the biologically active compound.

10. The method of any preceding claim, wherein there is dispersed in the first organic liquid a mixture of a membrane lipid and a micelle-forming lipid.

11. The method of claim 10, wherein the micelle forming lipid dispersed in the first organic liquid is a monoacyl lipid.

12. The method of any preceding claim, wherein the membrane lipid dispersed in the first organic liquid comprises egg yolk lecithin or soybean lecithin.

13. The method of claim 12, wherein the egg yolk lecithin or soybean lecithin is of a grade which comprises at least 40 wt% phosphatidyl choline.

14. A method according to any preceding claim, which comprises incorporating 2.5 - 25 % w/w of the first water-miscible organic liquid into said composition.

15. A method according to claim 14 wherein the first water-miscible organic liquid is incorporated in an amount of 2.5 - 10% w/w of the composition.

16. A method according to claim 16, wherein the first water-miscible organic liquid is incorporated in an amount of 5 - 7.5 wt% of the composition.

17. A method according to any of claims 14 to 16, wherein the first organic liquid is ethanol.

18. A method according to any of claims 14 to 16, wherein ethanol is present in the first organic liquid.

19. A method according to any of claims 14 to 18, which comprises incorporating 30 to 90% w/w of the second organic liquid into said composition.

20. A method according to any of claims 14 to 19, wherein the second organic liquid is incorporated in an amount of 40 to 90% w/w of the composition.

21. A method according to claim 21, wherein the second organic liquid is incorporated in an amount of 50 to 80 % w/w of the composition.

22. A method according to any of claims 14 to 21, wherein the second organic liquid comprises glycerol.

23. A method according to any preceding claim, further comprising incorporating into the composition a hydrocolloid, polymer or natural gum in an amount which is effective to increase the muco-adhesion of the composition.

24. A method according to claim 23, wherein there is incorporated 1 to 10% w/w of the hydrocolloid, polymer or natural gum.

25. A method according to claim 25, wherein there is incorporated 2 to 5% w/w of the hydrocolloid, polymer or natural gum.

26. A method according to any of claims 23 to 25, wherein the hydrocolloid or natural gum is a cellulose derivative, carrageenin or an alginate.

27. A method according to any preceeding claim, wherein the composition is produced is sterile.

## Patentansprüche

1. Verfahren zur Herstellung einer Zusammensetzung umfassend mindestens eine lipophile biologisch wirksame Verbindung in molekularer Dispersion in Doppelschichten einer im wesentlichen nicht-wässrigen Zusammensetzung umfassend mindestens ein Membranlipid, welches in einem hydrophilen Medium suspendiert ist, wobei das Verfahren die folgenden Schritte umfasst:
Lösen oder Dispergieren mindestens eines Teils des Membranlipids in einer ersten organischen Flüssigkeit, welche das Lipid lösen oder dispergieren kann;
Zusetzen der lipophilen biologisch wirksamen Verbindung zu der Lösung oder Dispersion; und
Zusetzen einer zweiten organischen Flüssigkeit, welche mit der ersten organischen Flüssigkeit mischbar ist und von solcher Natur ist und in einer solchen Menge vorliegt, dass das(die) Membranlipid oder -lipide mindestens teilweise in der Form von Doppelschichten vorliegt/vorliegen, wobei mindestens eine der ersten und zweiten Flüssigkeit mit Wasser vermischbar ist.

2. Verfahren nach Anspruch 1, wobei die biologisch wirksame Verbindung als ein mikronisiertes Pulver zu der Lösung oder Dispersion zugegeben wird.

3. Verfahren nach Anspruch 1 oder 2, wobei die zugegebene biologisch wirksame Verbindung ein Polyen-Makrolid ist.

4. Verfahren nach Anspruch 1 oder 2, wobei die zugegebene biologisch wirksame Verbindung Nystatin oder Amphotericin B ist.

5. Verfahren nach Anspruch 1 oder 2, wobei die zugegebene biologisch wirksame Verbindung ein Imidazolderivat ist.

6. Verfahren nach Anspruch 5, wobei die zugegebene biologisch wirksame Verbindung Clotrimazol oder Miconazol ist.

7. Verfahren nach Anspruch 6, wobei die zugegebene biologisch wirksame Verbindung 5-Fluorcystosin ist.

8. Verfahren nach einem der vorstehenden Ansprüche, wobei 0,1-2,5 % w/w der biologisch wirksamen Verbindung zugegeben werden.

9. Verfahren nach einem der Ansprüche 1 bis 8, wobei etwa 1 % w/w der biologisch wirksamen Verbindung zugegeben werden.

10. Verfahren nach einem der vorstehenden Ansprüche, wobei in der ersten organischen Flüssigkeit eine Mischung aus einem Membranlipid und einem micellenbildenden Lipid dispergiert wird.

11. Verfahren nach Anspruch 10, wobei das in der ersten organischen Flüssigkeit dispergierte micellenbildende Lipid ein Monoacyl-Lipid ist.

12. Verfahren nach einem der vorstehenden Ansprüche, wobei das in der ersten organischen Flüssigkeit dispergierte Membranlipid Eidotter-Lecithin oder Sojabohnen-Lecithin umfasst.

13. Verfahren nach Anspruch 12, wobei das Eidotter-Lecithin oder Sojabohnen-Lecithin von einer Güte ist, welche mindestens 40 Gew.-% Phosphatidylcholin umfasst.

14. Verfahren nach einem der vorstehenden Ansprüche, welches das Einbringen von 2,5-25 % w/w der ersten wassermischbaren organischen Flüssigkeit in die Zusammensetzung umfasst.

15. Verfahren nach Anspruch 14, wobei die erste wassermischbare organische Flüssigkeit in einer Menge von 2,5-10 % w/w der Zusammensetzung eingebracht wird.

16. Verfahren nach Anspruch 14, wobei die erste wassermischbare organische Flüssigkeit in einer Menge von 5-7,5 Gew.-% der Zusammensetzung eingebracht wird.

17. Verfahren nach einem der Ansprüche 14 bis 16, wobei die erste organische Flüssigkeit Ethanol ist.

18. Verfahren nach einem der Ansprüche 14 bis 16, wobei Ethanol in der ersten organischen Flüssigkeit vorliegt.

19. Verfahren nach einem der Ansprüche 14 bis 18, welches das Einbringen von 30 bis 90 % w/w der zweiten organischen Flüssigkeit in die Zusammensetzung umfasst.

20. Verfahren nach einem der Ansprüche 14 bis 19, wobei die zweite organische Flüssigkeit in einer Menge von 40 bis 90 % w/w der Zusammensetzung eingebracht wird.

21. Verfahren nach Anspruch 20, wobei die zweite organische Flüssigkeit in einer Menge von 50 bis 80 % w/w der Zusammensetzung eingebracht wird.

22. Verfahren nach einem der Ansprüche 14 bis 21, wobei die zweite organische Flüssigkeit Glycerin umfasst.

23. Verfahren nach einem der vorstehenden Ansprüche, ferner umfassend das Einbringen eines Hydrokolloids, Polymers oder Naturkautschuks in die Zusammensetzung in einer Menge, welche wirksam ist, um die Muko- bzw. Schleimhautadhäsion der Zusammensetzung zu erhöhen.

24. Verfahren nach Anspruch 23, wobei 1 bis 10 % w/w des Hydrokolloides, Polymers oder Naturkautschuks eingebracht werden.

25. Verfahren nach Anspruch 24, wobei 2 bis 5 % w/w des Hydrokolloides, Polymers oder Naturkautschuks eingebracht werden.

26. Verfahren nach einem der Ansprüche 23 bis 25, wobei das Hydrokolloid oder der Naturkautschuk ein Cellulosederivat, Carrageenin oder ein Alginat ist.

27. Verfahren nach einem der vorstehenden Ansprüche, wobei die Zusammensetzung, die hergestellt wird, steril ist.

## Revendications

1. Méthode de préparation d'une composition comprenant au moins un composé lipophile biologiquement actif dans une dispersion moléculaire en bicouches d'une composition substantiellement non aqueuse comprenant au moins un lipide membranaire suspendu dans un milieu hydrophile, ladite méthode incluant les étapes consistant à :
dissoudre ou disperser au moins une portion du lipide membranaire dans un premier liquide organique qui puisse dissoudre ou disperser le lipide.
ajouter le composé lipophile biologiquement actif à la solution ou dispersion et
ajouter un second liquide organique qui puisse être mélangé avec le premier liquide organique et qui soit d'une nature et dans une quantité telles que le lipide ou les lipides membranaire(s) soit ou soient au moins partiellement sous forme de bicouches, avec au moins un des premier et second liquides soulubles dans l'eau.

2. Méthode selon la revendication 1, dans laquelle le principe biologiquement actif est ajouté à la solution ou à la dispersion sous la forme de poudre micronisée.

3. Méthode selon la revendication 1 ou 2, dans laquelle le composé biologiquement actif ajouté est un macrolide polyène.

4. Méthode selon la revendication 1 ou 2, dans laquelle le composé biologiquement actif ajouté est de la nystatine ou de l'amphotéricine B.

5. Méthode selon la revendication 1 ou 2, dans laquelle le composé biologiquement actif ajouté est un dérivé d'imidazole.

6. Méthode selon la revendication 5, dans laquelle le composé biologiquement actif ajouté est un clotrimazole ou un miconazole.

7. Méthode selon la revendication 6, dans laquelle le composé biologiquement actif ajouté est de la 5-fluorocystosine.

8. Méthode selon l'une quelconque des revendications précédentes, dans laquelle on ajoute 0,1-2,5 % M/M du composé biologiquement actif.

9. Méthode selon les revendications 1 à 8, dans laquelle on ajoute environ 1 % M/M du composé biologiquement actif.

10. Méthode selon l'une quelconque des revendications précédentes, dans laquelle un mélange de lipide membranaire et de lipide sous forme de micelle est dispersé dans le premier liquide organique.

11. Méthode selon la revendication 10, dans laquelle le lipide formant micelle dispersé dans le premier liquide organique, est un lipide mono-acyle.

12. Méthode selon l'une quelconque des revendications précédentes, dans lequel le lipide membranaire dispersé dans le premier liquide organique comprend de la lécithine de jaune d'oeuf ou de la lécithine de soja.

13. Méthode selon la revendication 12, dans laquelle la lécithine de jaune d'oeuf ou la lécithine de soja sont d'une qualité qui comprend au moins 40 % en poids de phosphatidylcholine.

14. Méthode selon l'une quelconque des revendications précédentes, qui comprend l'incorporation de 2,5-25 % en poids du premier liquide organique soluble à l'eau dans ladite composition.

15. Méthode selon la revendication 14, dans laquelle le premier liquide organique soluble dans l'eau est incorporé en une quantité de 2,5 - 10 % M/M de la composition.

16. Méthode selon la revendication 14, dans laquelle le premier liquide organique soluble à l'eau dans une quantité de 5-7,5 % en poids de la composition.

17. Méthode selon l'une quelconque des revendications 14 à 16, dans laquelle le premier liquide organique est de l'éthanol.

18. Méthode selon l'une quelconque des revendications 14 à 16, dans laquelle l'éthanol est présent dans le premier liquide organique.

19. Méthode selon l'une quelconque des revendications 14 à 18, qui comprend l'incorporation de 30 à 90 % M/M du second liquide organique dans ladite composition.

20. Méthode selon l'une quelconque des revendications 14 à 19, dans laquelle le second liquide organique est incorporé en une quantité de 40 à 90 % M/M de la composition.

21. Méthode selon la revendication 20, dans laquelle le second liquide organique est incorporé en une quantité de 50 à 80 % M/M de la composition.

22. Méthode selon l'une quelconque des revendications 14 à 21, dans laquelle le second liquide organique comprend du glycérol.

23. Méthode selon l'une quelconque des revendications précédentes, comprenant en outre l'incorporation dans la composition d'un hycrocolloïde, d'un polymère ou d'une gomme naturelle en une quantité efficace pour augmenter la muco-adhésion de la composition.

24. Méthode selon la revendication 23, dans laquelle 1 à 10 % M/M d'hydrocolloïde, de polymère ou de gomme naturelle sont incorporés.

25. Méthode selon la revendication 24, dans laquelle 2 à 5 % M/M d'hydrocolloïde, de polymère ou de gomme naturelle sont incorporés.

26. Méthode selon l'une quelconque des revendications 23 à 25, dans laquelle l'hydrocolloïde ou la gomme naturelle est un dérivé de cellulose, de la carragénine ou un alginate.

27. Méthode selon l'une quelconque des revendications précédentes, dans laquelle la composition produite est stérile.
